# EUROPEAN PATENT APPLICATION

(11) **EP 1 142 987 A1**
(43) Date of publication of application: **10.10.2001**
(21) Application number: 00969856.4
(22) Date of filing: 18.10.2000
(51) Int. Cl.: C12N 1/20, C12N 1/04, A23L 1/30

(54) **COMPOSITION OF POWDERED AUREOBACIDIUM CULTURE SOLUTION, MANUFACTURING METHOD THEREOF AND POWDER MIXTURE WITH THE COMPOSITION**

(30) Priority: 19.10.1999 JP 29652999; 28.09.2000 JP 2000297248
(71) Applicant: Health Support Japan Corporation, Tokyo 101-0051 (JP)
(72) Inventor: NAKAHARA, Tetsuya, Chiba-shi, Chiba 262-0005 (JP); HYUGA, Makoto, Minato-ku, Tokyo 105-0014 (JP)
(74) Representative: Kyle, Diana
(86) International application number: JP0007235
(87) International publication number: WO0129184

(57) **Abstract**

Disclosed is a mixture excellent in coagulation of fats and oils together with a method of powdering the Aureobacidium culture solution. Formation of a powdered clathrate that is formed by including the Aureobacidium culture solution as a guest component into cyclodextrin as a host component improves its physical properties and shelf life. Preferably, chitosan is added to the above-described composition of the powdered Aureobacidium culture solution.

## Description

### TECHNICAL FIELD

The present invention relates to a composition of light yellow and viscous Aureobacidium culture solution as a food additive of which the physical properties and the shelf life are improved, a manufacturing method thereof and a powder mixture using the composition.

### BACKGROUND ART

Aureobacidium culture solution is a food additive involved in Notification No. 120 of the Ministry of Health and Welfare of Japan and prescribed that "it refers to a matter that includes β-1,3-1,6-glucan obtained from Aureobacidium as a principal component". This Aureobacidium culture solution is a culture solution that includes β-1,3-1,6-glucan that is produced commonly by subjecting a microbial strain of the Aureobacidium group to an aerobic liquid culture.

Attention has been focused on this sort of culture solution, being widely noticed as a food additive for health and especially expected of its carcinostatic effect. For example, the specification of Japanese Patent Publication No. Hei 3-48201 discloses the culturing method of a microbial strain of the Aureobacidium group, and polysaccharide substances, the culture product obtained therefrom, which includes glucose as the principal of the constitutional saccharides and show the bonding style consisting of β-1,3 principal bond chain and β-1,6 non-reducing terminal bond branch.

However, the Aureobacidium culture solution available at present becomes moldy depending on the storage condition because it is light yellow viscous liquid and apt to propagate bacteria therein, causing a problem that its long term storage is difficult. In an aspect of usage, there is another problem that handling it is troublesome due to its viscousness.

In the meantime, although the Aureobacidium culture solution has a coagulating property, the coagulation of its simple material is relatively weak. Thus, it is unable to coagulate oils such as fats.

The present invention has been accomplished in consideration of the above-mentioned problems, and a first object of which is to provide a powder material of Aureobacidium culture solution in order to improve the storability and handleability of Aureobacidium culture solution.

A second object of the present invention is to provide a mixture that includes a powder material of the Aureobacidium culture solution as a principal component which is superior in coagulating oils such as fats.

### DISCLOSURE OF THE INVENTION

Inventors of the present invention have hit upon an idea of powdering the viscous Aureobacidium culture solution, and after discussion, study, examination and evaluation on various techniques for the powdering found out that a clathration technique employing cyclodextrin as a host component gave a result better than expected for the object of the present invention, and reached to complete the present invention.

Thus, according to the present invention, there is provided a powder material of Aureobacidium, wherein the physical properties and shelf life of which were improved as the formation of powdered clathrate that Aureobacidium culture solution as a guest component and treated with cyclodextrin as a host component.

It is preferable to use as cyclodextrin, among α-, β-, and γ-cyclodextrin, that contains α-cyclodextrin 50 wt% or more.

The weight ratio of Aureobacidium culture solution and cyclodextrin is set commonly within a range from 20:1 to 5:1. When the viscosity of the mixture is too high, it is needed to adjust the viscosity value into an appropriate range by adding a proper quantity of water.

It is known that the Aureobacidium culture solution shows remarkable activity of coagulation for kaolin solution and reacts with aluminum ion quantitatively to form fiber flock. And aluminum sulfate is used as a typical substance to generate aluminum ion.

The present inventors have paid attention to the coagulating property of Aureobacidium and tried to separate fats and oils by utilizing the coagulation function.

However, subjects that the Aureobacidium culture solution can take remarkable coagulation effect are limited to substances like kaolin solution, not showing remarkable effect of coagulation on fats and oils.

However, it is confirmed that the powder material made by mixing Aureobacidium culture solution and cyclodextrin, when it is mixed into fats and oils together with aluminum sulfate, shows remarkable coagulation effect on fats and oils. This is considered to be an appearance of the effect that the excellent coagulating property of Aureobacidium culture solution and the clathration function of cyclodextrin worked together to coagulate fats and oils.

However, the aluminum sulfate mentioned above cannot be provided as is edible and cannot be stated harmless to the human body. Therefore, it is evident that aluminum sulfate is never a preferable substance to be included in soap, detergent and so on that are used to coagulate fats and oils content or the like and remove them.

Accordingly, while the present inventors made inquiry into substances that dissolve in water to generate a positive ion like aluminum sulfate and assist the coagulating property of the Aureobacidium culture solution, chitosan was able to be used in place of aluminum sulfate, that is a macromolecular saccharide and has a characteristic of becoming positively charged when dissolved in water.

As is well known, chitosan is typically an extract from a crab shell and is able to provide as is edible without any harm to the human body. This substance is a well-known additive in health foods. Its known effects include bringing down the blood pressure, preventing thrombus, enhancing immunocompetence, preventing propagation of bacteria or bacilli, having a (slimming) function of absorbing fats in ingested food and excreting it as it is, and having an effect of moisture retaining as cosmetics.

Based on the above-described knowledge, a powder mixture of cyclodextrin powder clathrating Aureobacidium culture solution (hereinafter simply referred to as "Aureobacidium culture solution powder") added with chitosan is provided in the embodiment of the present invention. It is preferable to set the mixing ratio of Aureobacidium culture solution powder and chitosan within a range from 10:1 to 1:5.

Conventionally, in order to show coagulating effect by using the Aureobacidium culture solution together with aluminum sulfate, a process such as adding Aureobacidium culture solution first and then adding aluminum sulfate into the solution was needed. However, when Aureobacidium culture solution was turned into a powder mixture being powdered by clathration of cyclodextrin and mixed with chitosan at the above-mentioned mixing ratio, it was possible to perform condensation of fats and oils by adding the powder mixture into fats and oils. This is considered that the large increase of the mixing ratio of chitosan to powdered Aureobacidium culture solution compared with the conventional one, from 100:1 to 20:1 is contributing.

The matter obtained by mixing Aureobacidium culture solution powder and chitosan in this way is expected to have excellent effects as a medical adjuvant or a health food, the effects of chitosan such as enhancing immunocompetence, bringing down blood pressure and preventing thrombus being added to the medical effects of Aureobacidium culture solution. Further, this powder mixture can be expected to have an excellent effect to remove internal fat due to addition of the slimming function by chitosan to the effect of the Aureobacidium culture solution powder to coagulate fats and oils.

Also, cosmetics, body shampoo and the like of good quality, which are made of these materials can be expected to have excellent effects because the moisture retaining effect of chitosan as cosmetics is added to the coagulating effect of the Aureobacidium culture solution powder.

According to a method of the present invention, a powdered clathration is obtained as described below. An appropriate quantity of cyclodextrin is added into Aureobacidium culture solution to obtain a liquefied mixture. Then, the obtained liquefied mixture is stirred to form a liquid mixture containing clathrates of the Aureobacidium culture solution as a guest component and the cyclodextrin as a host component. The liquid mixture containing the clathrates is subject to spray drying to obtain a powdered clathrate.

The stirring process is preferably performed as vigorous agitation using a high shear stirrer, for example, a homogenizer. The stirring time depends on the strength of agitation, however, a proper clathration process can be typically performed with the extent of the speed of stirrer revolution at 500-10000 rpm for 10-90 minutes, preferably at 2000 rpm for 20-40 minutes. Further, the application of ultrasonic to the mixture under agitation during at least a part, preferably the full, of the time of this stirring process gives a preferred result in the improvement in the operation efficiency, promoting the clathration process remarkably.

After a stirring process in this way is finished, the mixture is subjected to the spray drying treatment. The temperature of the spray drying is within the range of about 150°C -180°C, preferably 160°C-175°C, according to the study in terms of both drying efficiency and preventing degradation of the product.

The powder obtained by the spray drying shows free fluidity and provides a good handleability. Therefore, the weighing work is facilitated. Also, tableting tests of the powder added with a proper excipient and a lubricant at need indicated that proper strength and shape holding property for various sizes could be attained. Therefore, tablets with a size suitable for taking by mouth can be produced. Further, it was also demonstrated that satisfiable products could be obtained by tableting with no excipient for common uses. Also, in a long-term standing storage test, the far longer period of time had passed until recognizing the early growth of mold for the product compared with the control material of untreated Aureobacidium culture solution.

### BEST MODE FOR CARRYING OUT THE INVENTION

### <EXAMPLE 1>

Mixture of 700 parts by weight of a commercially available Aureobacidium culture solution ("BAKUSHO"™: from Health Support Japan Corporation: Tokyo) and 300 parts by weight of cyclodextrin ("K-100"™: from Ensuiko Sugar Refining Co., Ltd.) which contains about 70 wt% α-cyclodextrin by weight was mixed with 1000 parts by weight of water and stirred vigorously with a homogenizer. The stirring condition was set at 4000 rpm for the first 10 minutes and 2000 rpm for the subsequent 15 minutes. Through this stirring process, clathration was performed containing the Aureobacidium culture solution as a guest into the central cavity of cyclodextrin as a host. After the stirring process was finished, the liquid mixture was spray-dried with a spray dryer and the like at about 170°C and a composition of powdered Aureobacidium culture solution was obtained. The powder showed a sufficient coagulating effect when it was used together with aluminum sulfate.

Next, experiments on powders in the case where the Aureobacidium culture solution was mixed with α-CD, β-CD and γ-CD respectively were carried out. Each powder in which α-CD, β-CD and γ-CD were used under the same conditions as those of the above-described EXAMPLE 1 respectively was used together with aluminum sulfate and examined whether or not coagulating function was achieved. The result is shown in TABLE 1. In TABLE 1, "G" for "Good" represents the case where coagulating function was recognized, and "NG" for "Not Good" represents the case where the coagulation function was slightly recognized, but not remarkably.

**TABLE 1**

| | | | |
|---|---|---|---|
| Aureobacidium culture solution | α-CD | β-CD | γ-CD |
| Evaluation of powdering | G | NG | NG |

Then, α-CD was mixed with the mixture of β-CD and γ-CD (1:1) and coagulating property in the case where only the content ratio of α-CD was varied was examined. The result is shown in TABLE 2.

**TABLE 2**

| α-CD | (β-CD) + (γ-CD) | Evaluation of powdering |
|---|---|---|
| 10(wt%) | 90(wt%) | NG |
| 30 | 70 | NG |
| 50 | 50 | G |
| 70 | 30 | G |
| 80 | 20 | G |

According to the above results, powdering with good coagulation is made possible when the Aureobacidium culture solution is mixed with cyclodextrin containing 50 wt% or more α-CD.

### =Stability Test=

### (1)Right after Production

In order to check the stability holding of compositions of the Aureobacidium culture solution in the form of powdered clathration produced by the method of the present invention, the property of rapid coagulation (gelling) by aluminum ion in the culture solution described in the above-mentioned Patent Office Gazette was utilized.

20 parts by weight of the composition of powdered Aureobacidium culture solution (sample A) obtained by the present example was mixed with 100 parts of water, then stirred well to a state of solution. When 20 parts by weight of 25 wt% aqueous solution of aluminum sulfate was added herein, the solution gelled at once and turned into paste like "konjak" jelly. For comparison, the same test was repeated for 20 parts of the raw material of Aureobacidium culture solution (sample B: control) used for the present example, and the similar gelling was observed.

Accordingly, the result shows that the culture solution contents are substantially held stable and do not substantially receive any change in quality like deterioration, degradation and so on through the treatments of clathration and spray drying due to the present invention.

### (2) After Storage

The same samples A and B mentioned above were subjected to the same test described as in (1) above after they had stored as they were in wide-mouthed bottles being opened in a room from spring to summer, for three months from the middle of July to the middle of September (in Tokyo). The sample A of the present invention showed a gel formation with the hardness equal to that of a fresh one. As for the sample B, it started to become moldy on the surface after a week of storage, and became covered with mold over the whole surface giving out a nasty smell in two weeks. It was not a usable condition, and it was clear that the contents had changed in quality, deteriorated or degraded. As for the sample A based on the present invention, a slight growth of mold was observed on the surface portion just before the end of the examination period, but it did not extend all over, and no nasty smell generation was recognized.

### <EXAMPLE 2>

The operation of EXAMPLE 1 was herein employed. In addition to the operation, the amounts of Aureobacidium culture solution and cyclodextrin used in EXAMPLE 1 were changed to 900 parts by weight and 100 parts by weight respectively, and ultrasonic of 90 kHz (1 kW nominal power) was applied during the latter part (2000 rpm for 15 minutes) of the stirring process. The composition obtained here gelled extremely hard in the stability test. Except for that, about the same result as that of EXAMPLE 1 in terms of the stability tests (1) and (2) as well as the tableting test was obtained.

### <EXAMPLE 3>

### (1) Coagulation Test of Fats and Oils

100 ml of water in a beaker was added with 3 mg of a Chinese chili oil and stirred well. Then each 10 mg of (i) Aureobacidium culture solution, (ii) cyclodextrin, (iii) powder of cyclodextrin clathrating Aureobacidium culture solution, and (iv) chitosan (product of Koyo Chemical Co., Ltd), was added as a simple material or a mixture of them and stirred. Then, states of coagulation of the chili oil were observed. Note that in the case of above mixture, the mixing ratio was set equal. Coagulation of the chili oil into a spherical shape with a diameter of 3 mm or more was evaluated as "G", coagulated sphere with a diameter of more than 2 mm to less than 3 mm was evaluated as "M" for "Moderate", and coagulated sphere with a diameter of less than 2 mm was evaluated as "NG." The results are shown in TABLE 3. The reason why the chili oil was used in the experiments here is that the chili oil has a red color and easy to confirm visually the existence of coagulation.

**TABLE 3**

| | Exp.1 | Exp.2 | Exp.3 | Exp.4 | Exp.5 | Exp.6 | Exp.7 | Exp.8 | Exp.9 |
|---|---|---|---|---|---|---|---|---|---|
| (i) | add | | | | add | add | add | | |
| (ii) | | add | | | add | | add | add | |
| (iii) | | | add | | | | | | add |
| (iv) | | | | add | add | add | | add | add |
| Mark | NG | NG | NG | NG | M | M | NG | NG | G |

Next, experiments on coagulation were conducted for a variety of mixing ratios of chitosan. In these experiments, coagulation was evaluated in the same manner as described above by varying only adding amount of the powder of cyclodextrin clathrating Aureobacidium culture solution (CD powder) for various values to 10 mg of chitosan. The results are shown in TABLE 4.

**TABLE 4**

| | | | | | | | | | |
|---|---|---|---|---|---|---|---|---|---|
| Chitosan(mg) | 10.0 | 10.0 | 10.0 | 10.0 | 10.0 | 10.0 | 10.0 | 10.0 | 10.0 |
| CD powder(mg) | 0.5 | 0.8 | 1.0 | 2.5 | 5.0 | 10.0 | 50.0 | 75.0 | 100 |
| Mark | NG | M | G | G | G | G | G | M | NG |

From the results above, it was proved that good coagulation of fats and oils could be obtained when chitosan was added to the powder of cyclodextrin clathrating Aureobacidium culture solution (CD powder) within the range of 10:1 to 1:5.

Although cases of using the chili oil among fats and oils are shown in the above-mentioned examples of experiment, the present invention was able to get the same results in the cases of using other oils such as a sesame oil, a salad oil and so on.

### INDUSTRIAL APPLICABILITY

The Aureobacidium culture solution composition according to the present invention has improved physical properties and shelf life in the form of powdered clathrations formed by treating the Aureobacidium culture solution as a guest component with cyclodextrin as a host component. Converting a viscous liquid of the usual Aureobacidium culture solution into a powder both improves the storability and handleability, and extends its application field and development area widely.

Meanwhile, because the powder mixture of the composition of powdered Aureobacidium culture solution added with chitosan shows remarkable coagulation effect on fats and oils and chitosan is an edible matter, the mixture is also excellent in usefulness that it can be expected possibility of being used as a material of not only soap, cosmetics and the like but also for medicines, health foods and slimming foods.

## Claims

1. A composition of Aureobacidium culture solution of improved physical properties and shelf life, which is in the form of powdered clathrates in which Aureobacidium culture solution as a guest component is included in cyclodextrin as a host component.

2. A composition of Aureobacidium culture solution claimed in claim 1, wherein the cyclodextrin contains a-cyclodextrin of 50 wt% or more among α-, β-, and γ-cyclodextrin.

3. A manufacturing method of Aureobacidium culture solution powder comprising the following steps of:
adding an appropriate quantity of cyclodextrin into Aureobacidium culture solution to obtain a liquefied mixture;
stirring the liquefied mixture to form a liquid mixture containing clathrates of the Aureobacidium culture solution as a guest component and the cyclodextrin as a host component; and
spray drying the liquid mixture into a powdered clathrate.

4. A method claimed in claim 3, wherein the liquid mixture is applied with ultrasonic in the stirring step.

5. A method of any one of claims 3 or 4, wherein a weight ratio of the Aureobacidium culture solution to the cyclodextrin is within the range of 20:1 to 5:1.

6. A powder mixture wherein chitosan is added to the composition of powdered Aureobacidium culture solution of any one of claims 1 or 2.

7. A powder mixture claimed in claim 6, wherein the mixing weight ratio of the composition of the powdered Aureobacidium culture solution to chitosan is set from 10:1 to 1:5.
